(19) Europäisches Patentamt
European Patent Office
Office européen des brevets

(11) Veröffentlichungsnummer: **0 166 172**
**B1**

(12) **EUROPÄISCHE PATENTSCHRIFT**

(45) Veröffentlichungstag der Patentschrift: **05.10.88**

(51) Int. Cl.⁴: **C 07 D 229/00,** C 08 G 18/79

(21) Anmeldenummer: **85106052.5**

(22) Anmeldetag: **17.05.85**

(54) **Neue Uretdiondiisocyanate, diese Diisocyanate enthaltende Polyisocyanatgemische, ein Verfahren zu deren Herstellung und ihre Verwendung zur Herstellung von Polyurethankunststoffen.**

(30) Priorität: **30.05.84 DE 3420113**

(43) Veröffentlichungstag der Anmeldung: **02.01.86 Patentblatt 86/1**

(45) Bekanntmachung des Hinweises auf die Patenterteilung: **05.10.88 Patentblatt 88/40**

(84) Benannte Vertragsstaaten: **BE DE FR GB IT NL**

(56) Entgegenhaltungen:
**DE - B - 1 230 425**
**US - A - 2 671 082**
**US - A - 2 683 144**

(73) Patentinhaber: **BAYER AG, Konzernverwaltung RP Patentabteilung, D-5090 Leverkusen 1 Bayerwerk (DE)**

(72) Erfinder: **Scholl, Hans-Joachim, Dr., Rudolf-Sohm-Strasse 28, D-5000 Koeln 60 (DE)**
Erfinder: **Pedain, Josef, Dr., Haferkamp 6, D-5000 Koeln 80 (DE)**

**EP 0 166 172 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

AOTORUM AG

**Beschreibung**

Die vorliegende Erfindung betrifft neue Uretdiondiisocyanate enthaltende Polyisocyanatgemische, ein Verfahren zu ihrer Herstellung durch teilweise Dimerisierung der Isocyanatgruppen von speziellen $C_8$-$C_{15}$-Alkyl-substituierten Phenylendiisocyanaten und ihre Verwendung als Isocyanatkomponente bei der Herstellung von Polyurethankunststoffen.

Die Dimerisierung von organischen Isocyanaten unter Bildung von Uretdiongruppen ist seit langem bekannt. So beschreibt beispielsweise die US-A 2 671 082 die Dimerisierung unter Verwendung von speziellen aromatisch-aliphatischen tertiären Phosphinen als Dimerisierungskatalysatoren. Die US-A 2 683 144 ihrerseits beschreibt die Dimerisierung von organischen Isocyanaten in Gegenwart von tertiären Phosphin-Katalysatoren, wobei man zur Abstoppung der Reaktion, d.h. als Katalysatorengift bestimmte Alkylierungsmittel verwendet. In den Ausführungsbeispielen dieser beiden Patente werde vor allem Monoisocyanate dimerisiert. Soweit Diisocyanate als Ausgangsmaterialien eingesetzt werden, stellen die Umsetzungsprodukte hochschmelzende Festkörper dar. Eine Literaturzusammenfassung betreffend die Herstellung von Uretdiongruppen aufweisenden Polyisocyanaten findet sich beispielsweise in «Small Ring Heterocyclus» Part 2, Edited by Alfred Hassner, John Wiley and Sons, New York, (1983) S. 522 ff. Uretdiongruppen aufweisende Polyisocyanate sind interessante Aufbaukomponenten für die Polyurethanchemie, da sie sowohl freie als auch in Form der Uretdiongruppen blockiert vorliegende Isocyanatgruppen aufweisen. So ist es beispielsweise möglich, Uretdiongruppen aufweisende Polyisocyanate über die freien Isocyanatgruppen inaktive Wasserstoffatome aufweisende Kunststoffvorläufer einzubauen, die dann beim Erhitzen durch Reaktion der in Form der Uretdiongruppen blockiert vorliegenden Isocyanatgruppen mit den aktiven Wasserstoffatomen in den vernetzten Zustand übergehen. Uretdiongruppen aufweisende Polyisocyanate mit Blockierungsmittel für Isocyanatgruppen blockierten Isocyanatgruppen stellen insofern besonders interessante blockierte Polyisocyanate dar, als die Gesamtkonzentration an flüchtigen Blockierungsmitteln im Vergleich zu den üblichen blockierten Polyisocyanaten wesentlich geringer ist, da die Uretdiongruppen selbst Blockierungsmittel-freie, blockierte Isocyanatgruppen darstellen.

Zur Herstellung der Uretdiongruppen aufweisenden Polyisocyanate werden die unterschiedlichsten Katalysatoren eingesetzt. In neuerer Zeit haben sich insbesondere Phosphinderivate und Pyridin-Derivate wie 4-Dialkylaminopyridine als Dimerisierungskatalysatoren bewährt.

Den meisten bekannten Uretdiongruppen aufweisenden Polyisocyanaten haften eine Reihe verbesserungswürdige Nachteile an, die ihre technische Verwendung verhindern oder stark einschränken.

So enthalten Uretdiongruppen aufweisende aromatische Polyisocyanate schwerlösliche Festkörperanteile. Andere Uretdiongruppen aufweisende Polyisocyanate des Standes der Technik sind mit unpolaren Lacklösungsmitteln wie z.B. aliphatischen oder aromatischen Kohlenwasserstoffen nur schlecht verdünnbar. Dieser Nachteil äussert sich darin, dass beim Vermischen der Uretdiongruppen enthaltenden Polyisocyanate mit aliphatischen oder aromatischen Kohlenwasserstoffen bzw. mit Polyestern, Polyacrylaten oder Alkydharzen, die in diesen Lösemitteln gelöst sind, Trübungen und Ausfällungen auftreten, die z.B. die Herstellung von Lacken unmöglich machen.

Ein weiterer Nachteil der Uretdiongruppen aufweisenden Polyisocyanate des Standes der Technik ist auf deren oft anzutreffende Unverträglichkeit mit anderen Bindemitteln und Reaktionspartnern zurückzuführen, die sich in einer oft zu kurzen Verarbeitungszeit der gebrauchsfertigen Lackmischungen äussert. Der Grund für diese oft zu kurze Verarbeitungszeit ist insbesondere darauf zurückzuführen, dass bei Ausbildung von nur wenigen Urethangruppen in den gebrauchsfertigen Lacken die ohnehin geringe Löslichkeit bzw. Verträglichkeit noch schlechter wird und das Bindemittel aus der Lacklösung ausfällt. Der naheliegende Gedanke, die schlechte Verträglichkeit durch Mitverwendung von polaren Lösungsmitteln zu beseitigen, führt zu einer beträchtlichen Verteuerung der Lacke. Die in der DE-AS 2 414 413 empfohlene Modifizierung der Polyisocyanate mit Fettalkoholen beinhaltet eine wesentliche Verbesserung, ist jedoch mit dem Nachteil behaftet, dass durch diese Modifizierung Isocyanatgruppen verbraucht werden, die nicht mehr zur Ausbildung von Vernetzungsstellen zur Verfügung stehen und die Funktionalität der Polyisocyanate vermindern.

Es war daher die Aufgabe der vorliegenden Erfindung, neue, auf einfachem Wege zugängliche Uretdiongruppen aufweisende Polyisocyanate zur Verfügung zu stellen, die keine festen Bestandteile aufweisen, die mit wenig polaren Löse- und Verdünnungsmitteln verträglich und mit Hydroxyl-Polyestern, -Polyacrylaten und -Alkydharzen ohne Eintrübung auch in Gegenwart von unpolaren Lösemitteln mischbar sind und die schliesslich im Gemisch mit diesen Reaktionspartnern eine verbesserte, möglichst lange Verarbeitungszeit aufweisen.

Diese Aufgabe konnte mit der Bereitstellung der nachstehend näher beschriebenen Polyisocyanatgemische bzw. des Verfahrens zu ihrer Herstellung gelöst werden.

Gegenstand der Erfindung sind bei Raumtemperatur flüssige, gegebenenfalls in Abmischung mit Hilfs- und Zusatzmitteln der bei der Herstellung von Polyurethankunststoffen mitverwendeten Art vorliegende Polyisocyanatgemische, dadurch gekennzeichnet, dass sie, bezogen auf die Summe a + b + c,

a) zu 10 bis 80 Gew.% aus Uretdiondiisocyanaten der Formel I

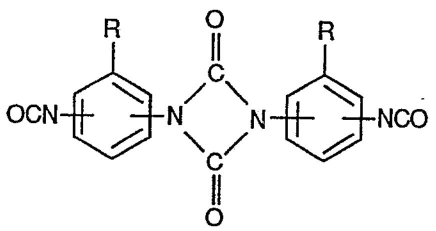

I

für welche R jeweils für einen $C_8$-$C_{15}$-Alkalyrest steht,

b) zu 0 bis 20 Gew.% aus höheren Homologen der unter a) genannten Diisocyanate mit mehr als einem Uretdionring und

c) zu 20 bis 90 Gew.% aus Diisocyanaten der Formel II

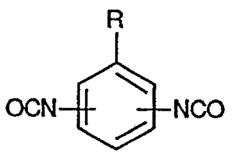

II

bestehen, wobei R die genannte Bedeutung hat und wobei sich die Prozentsätze jeweils zu 100 ergänzen.

In den erfindungsgemässen Polyisocyanatgemischen liegen die Uretdiondiisocyanate, herstellungsbedingt, als Lösung in den zu ihrer Herstellung eingesetzten Ausgangsdiisocyanaten vor, wobei die erfindungsgemässen Gemische noch ausserdem geringe Mengen an höheren Homologen der erfindungsgemässen Diisocyanate aufweisen können.

Gegenstand der Erfindung ist auch ein Verfahren zur Herstellung dieser Polyisocyanatgemische durch Dimerisierung eines Teils der Isocyanatgruppen von aromatischen Diisocyanaten in Gegenwart von Dimerisierungskatalysatoren und Abbruch der Dimerisierungsreaktion beim jeweils gewünschten Dimerisierungsgrad durch Zugabe eines Katalysatorengifts, dadurch gekennzeichnet, dass man als zu dimerisierende Diisocyanate gegebenenfalls als Homologen- und/oder Isomerengemisch vorliegende Diisocyanate der obengenannten Formel II verwendet.

Gegenstand der Erfindung ist schliesslich auch die Verwendung dieser Polyisocyanatgemische gegebenenfalls in Abmischung mit anderen organischen Polyisocyanaten und gegebenenfalls in mit Blockierungsmitteln für Isocyanatgruppen blockierter Form als Isocyanatkomponente bei der Herstellung von Polyurethankunststoffen nach dem Isocyanat-Polyadditionsverfahren.

Als erfindungswesentliche Ausgangskomponente werden Diisocyanate der bereits genannten allgemeinen Formel eingesetzt, für welche R die bereits obengenannte Bedeutung hat. Vorzugsweise werden solche derartige Polyisocyanate eingesetzt, die als Homologen- und Isomerengemisch vorliegen, und für welche R für einen gesättigten, geradkettigen, aliphatischen Kohlenwasserstoffrest mit 8 bis 15, insbesondere 10 bis 13 Kohlenstoffatomen steht. Die Herstellung dieser bevorzugt als erfindungswesentliche Ausgangskomponente einzusetzenden Diisocyanate ist beispielsweise in der EP-A 0 058 368 bzw. der DE-OS 3 105 776 beschrieben. Insbesondere die in der genannten Europäischen Veröffentlichung beschriebenen Diisocyanate stellen die erfindungsgemäss bevorzugten Ausgangsmaterialien dar. Ausser diesen bevorzugten Ausgangsmaterialien können beim erfindungsgemässen Verfahren als Ausgangskomponenten auch solche Diisocyanate der genannten allgemeinen Formel eingesetzt werden, für welche R für einen verzweigten aliphatischen Kohlenwasserstoffrest mit 8 bis 15 Kohlenstoffatomen steht, und wie sie beispielsweise gemäss US-PS 2 986 576 zugänglich sind.

Beim erfindungsgemässen Verfahren können beliebige literaturbekannte Katalysatoren für die Dimerisierung von aromatischen Isocyanaten eingesetzt werden. Besonders gut geeignet sind beispielsweise Aminopyridine wie z.B. 4-(1-Pyrrolidinyl)-pyridin oder 4-(Dimethylamino)-pyridin oder Phosphinderivate wie z.B. Tris (N,N-dimethyl-amino)-phosphin, die vorzugsweise in hydroxylgruppenhaltigen Verbindungen wie 2-Ethylhexanol oder Polyethylenglykol gelöst zum Einsatz gelangen.

Der Abbruch der erfindungsgemässen Dimerisierungsreaktion erfolgt durch Zugabe von Katalysatorgiften literaturbekannter Art, wie z.B. anorganischen oder organischen Säuren oder Alkylierungsmitteln. Bevorzugt werden Säuren wie organische Sulfonsäuren oder Phosphorsäuredialkylester verwendet.

Das erfindungsgemässe Verfahren wird vorzugsweise lösungsmittelfrei durchgeführt, kann jedoch auch in Anwesenheit von inerten Lösungs- und Verdünnungsmitteln durchgeführt werden. Als inerte Lösungsmittel können unpolare Verdünnungsmittel wie Toluol, Xylol, höhere Aromaten, Leichtbenzin, Testbenzin und $C_{12}$-$C_{20}$-Alkyl-sulfonsäureester, aber auch inerte polare Lösungsmittel wie Ester und Ketone bzw. Gemische derartiger Lösungsmittel eingesetzt werden.

Die erfindungsgemässe Dimerisierungsreaktion wird im allgemeinen im Temperaturbereich zwischen 0 und 60°C, vorzugsweise 10 bis 40°C durchgeführt.

Die erfindungsgemässe Dimerisierungsreaktion wird im allgemeinen bei Erreichen eines Dimerisierungsgrads (Dimerisierungsgrad = Prozentsatz der dimerisierten Isocyanatgruppen, bezogen auf Gesamtmenge der im Ausgangspolyisocyanat vorliegenden Isocyanatgruppen) von 5 bis 40 vorzugsweise 10 bis 35% abgebrochen. Der Verlauf der Reaktion kann z.B. durch fortlaufende Bestimmung des Brechungsindex' verfolgt werden.

Je nach Dimerisierungsgrad entstehen beim erfindungsgemässen Verfahren bei Raumtemperatur flüssige Gemische mit unterschiedlichem Gehalt an den obengenannten Einzelkomponenten a), b) und c). Vorzugsweise weisen die erfindungsgemässen Gemische, bezogen auf die Summe a + b + c,

a) 20 bis 70 Gew.% an Uretdiondiisocyanaten der Formel I

b) 0 bis 10 Gew.% an höheren, mehr als einen Uretdionring aufweisenden Homologen der unter a) genannten Uretdiondiisocyanate und

c) 30 bis 80 Gew.% an Ausgangsdiisocyanaten der Formel II auf.

Diese erfindungsgemässen Polyisocyanatgemische sind bei Raumtemperatur, d.h. bei 20°C flüssig. Die erfindungsgemässen Gemische können auch in Abmischung mit den aus der Polyurethanchemie bekannten Hilfs- und Zusatzmitteln vorliegen. Hierzu gehören insbesondere die gegebenenfalls bei ihrer Herstellung mitverwendeten inerten Lösungsmittel.

Der erfindungswesentliche Punkt ist in dem Umstand zu sehen, dass aufgrund der erfindungsgemässen Auswahl der beim erfindungsgemässen Verfahren einzusetzenden Ausgangsdiisocyanate Uretdiongruppen aufweisende Diisocyanate entstehen, die in Form von klaren Lösungen in den Ausgangsdiisocyanaten vorliegen, ohne dass es zu Kristallisationsproblemen kommt.

Die erfindungsgemässen Polyisocyanatgemische können selbstverständlich in an sich bekannter Weise mit geeigneten Blockierungsmitteln für Isocyanatgruppen wie z.B. Phenol, ε-Caprolactam, Malonsäurediethylester oder Acetessigsäureethylester blockiert werden.

Die erfindungsgemässen Verfahrensprodukte bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar. Sie eignen sich insbesondere als Isocyanatkomponente in Zweikomponentenpolyurethanlacken. Bei der erfindungsgemässen Verwendung der erfindungsgemässen Polyisocyanatgemische können diese selbstverständlich auch in Abmischung mit weiteren organischen Polyisocyanaten zum Einsatz gelangen. Geeignete Abmischkomponenten sind beispielsweise die technisch wichtigen aromatischen Polyisocyanate wie 2,4- und 2,6-Diisocyanatotoluol und insbesondere 4,4-Diisocyanato-diphenylmethan bzw. flüssige Di- und Polyisocyanate auf Basis dieses in reiner Form festen Diisocyanats.

Die folgenden Beispiele erläutern die Erfindung. Alle Prozentangaben betreffen, soweit nicht anderslautend ausgeführt, Gewichtsprozente. Alle Angaben in «Teilen» betreffen Gewichtsteile. Die Analyse der Zusammensetzung der Umsetzungsprodukte erfolgte gelchromatographisch.

Beispiele

In den nachfolgenden Beispielen werden folgende Ausgangsmaterialien bzw. Hilfsmittel eingesetzt:

Diisocyanat I:
Diisocyanatgemisch gemäss Beispiel 1 der EP-A 0 058 368, NCO-Gehalt (gefunden): 25,5%, Siedebereich bei 2,2 mbar: 185 bis 203°C.

Katalysator A:
Lösung von 1 Teil 4-(1-Pyrrolidinyl)-pyridin in 1 Teil 2-Ethyl-1-hexanol.

Katalysator B:
Lösung von 1 Teil 4-(Dimethylamino)-pyridin in 9 Teilen technischem Octaethylenglykol.

Katalysator C:
Lösung von 1 Teil Tris(N,N-dimethylamino)phosphin in 1 Teil technischem Octaethylenglykol.

Abstopper A:
«Marlon® $AS_3L$» eine handelsübliche Alkylbenzolsulfonsäure der Fa. Chemische Werke Hüls AG.

Abstopper B:
Phosphorsäure-bis-(2-ethylhexylester).

Beispiel 1

100 Teile Diisocyanat I und 0,3 Teile Katalysator A werden bei 20°C gerührt, die rasch einsetzende Dimerisierung wird bei einem Brechungsindex von $n_D^{23°C}$: 1.5306 (Ausgangswert = $n_D^{23°C}$ : 1.5165) nach 2 Stunden mit 0,8 Teilen Abstopper A versetzt und 30 Minuten nachgerührt.

Man erhält ein Uretdiongruppen aufweisendes Polyisocyanat-Gemisch als klare Lösung mit den Daten: $n_D^{23°C}$ : 1.5307; NCO-Gehalt: 19,3%.

Die Lösung weist einen Gehalt von 42% an nicht umgesetztem Diisocyanat I auf. Die Uretdiongruppen aufweisenden Diisocyanate bestehen laut gelchromatographischem Befund zu ca. 90% aus Uretdiondiisocyanaten der Formel I mit einem Uretdionring und zum Rest aus höheren, mehr als einen Uretdionring aufweisenden Homologen.

Beispiele 2–5

Entsprechend Beispiel 1 wurden die Beispiele 2 bis 5 unter Verwendung von Diisocyanat I als Ausgangsdiisocyanat durchgeführt. Die Ergebnisse sind in der Tabelle I zusammengefasst.

Tabelle I

| Beispiel | Reaktions-Temperatur | Reaktions-Zeit | Katalysator A (Teile) | NCo (%) | $n_D^{23°C}$ |
|---|---|---|---|---|---|
| 2 | 20°C | 5 Min. | 0,4 | 23,0 | 1.5228 |
| 3 | 20°C | 15 Min. | 0,4 | 21,6 | 1.5250 |
| 4 | 20°C | 40 Min. | 0,4 | 20,7 | 1.5275 |
| 5 | 20°C | 90 Min. | 0,4 | 20,1 | 1.5290 |

Beispiel 6

100 Teile Diisocyanat I und 1 Teil Katalysator B werden 1 Tag bei Raumtemperatur stehen gelassen. Danach ist der Brechungsindex auf $n_D^{21^\circ C}$ : 1.5302 gestiegen. Man stoppt mit 0,4 Teilen Abstopper A und erhält ein Uretdiongruppen aufweisendes Polyisocyanatgemisch als klare Lösung mit den Daten: $n_D^{21^\circ C}$ : 1.5304; NCO-Gehalt: 19,5%.

Die Lösung weist einen Gehalt an unverändertem Ausgangsdiisocyanat I von 48% auf. Der Gehalt der Uretdiongruppen aufweisenden Polyisocyanate an Uretdiondiisocyanat der Formel I (ein Uretdionring) liegt, bezogen auf die Gesamtmenge an Uretdiongruppen aufweisenden Polyisocyanaten, bei ca. 92%.

Beispiel 7

100 Teile Diisocyanat I und 0,5 Teile Katalysator B werden 4 Stunden bei 20°C gerührt. Danach ist der Brechungsindex auf $n_D^{23^\circ C}$ : 1.5246 angestiegen. Man stoppt mit 0,3 Teilen Abstopper B ab und erhält ein Uretdiondiisocyanat enthaltendes Polyisocyanatgemisch in Form einer klaren Lösung mit den Daten $n_D^{23^\circ C}$ : 1.5247; NCO-Gehalt: 22,0%.

Der Gehalt des Gemisches an unverändertem Diisocyanat I liegt bei 69%. Der Gehalt der in dem Gemisch vorliegenden Uretdionpolyisocyanate an Uretdiondiisocyanat der Formel I liegt bei ca. 97%.

Beispiel 8

100 Teile Diisocyanat I und 0,3 Teile Katalysator A werden 3 h bei 20°C gerührt. Danach ist der Brechungsindex auf $n_D^{23^\circ C}$ : 1.5309 gestiegen. Man stoppt mit 0,8 Teilen Abstopper B und erhält ein Uretdiondiisocyanate und überschüssiges Diisocyanat I aufweisendes Polyisocyanat in Form einer klaren Lösung mit den Daten: $n_D^{23^\circ C}$ : 1.5310; NCO-Gehalt: 19,1%.

Der Gehalt des Gemisches an unumgesetztem Diisocyanat I liegt bei 38%.

Beispiel 9

75 Teile Diisocyanat I und 25 Teile Xylol werden gemischt und mit 0,3 Teilen Katalysator A versetzt. Man rührt 20 Minuten bei 20°C. Danach ist der Brechungsindex auf $n_D^{23^\circ C}$ : 1.5189 gestiegen. Nach Zusatz von 0,5 Teilen Abstopper B erhält man eine 75%ige Lösung in Xylol eines Uretdiondiisocyanate aufweisenden Polyisocyanatgemisches mit den Daten: $n_D^{23^\circ C}$ : 1,5190; NCO-Gehalt: 15,1%. Der Gehalt an nicht umgesetztem Diisocyanat I liegt bei 52%.

Beispiel 10

100 Teile Diisocyanat I und 0,4 Teile Katalysator C werden 90 Minuten bei 15°C gerührt. Danach ist der Brechungsindex auf $n_D^{23^\circ C}$ : 1.5307 gestiegen. Man stoppt mit 0,7 Teilen Abstopper B und erhält ein Uretdiondiisocyanat und überschüssiges Diisocyanat I aufweisendes Polyisocyanat in Form einer klaren Lösung mit den Daten: $n_D^{23^\circ C}$ : 1.5308; NCO-Gehalt: 19,1%. Der Gehalt des Gemisches an unumgesetztem Diisocyanat I liegt bei 40%.

Beispiel 11

100 Teile Diisocyanat I und 0,2 Teile Katalysator C werden 2 Stunden bei 20°C gerührt. Danach ist der Brechungsindex auf $n_D^{23^\circ C}$ : 1.5268 gestiegen. Man stoppt mit 0,4 Teilen Abstopper B und erhält ein Uretdiondiisocyanat und überschüssige Diisocyanat I aufweisendes Polyisocyanat in Form einer klaren Lösung mit den Daten: $n_D^{23^\circ C}$ : 1.5270; NCO-Gehalt: 20,9%.

Der Gehalt des Gemisches an nicht umgesetztem Diisocyanat I liegt bei 61%.

**Patentansprüche**

1. Bei Raumtemperatur flüssige, gegebenenfalls in Abmischung mit Hilfs- und Zusatzmitteln der bei der Herstellung von Polyurethankunststoffen mitverwendeten Art vorliegende Polyisocyanatgemische, dadurch gekennzeichnet, dass sie, bezogen auf die Summe a + b + c,
a) zu 10 bis 80 Gew.% aus Uretdiondiisocyanaten der Formel I

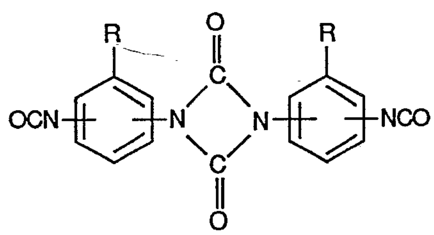 I

für welche R jeweils für einen $C_8$-$C_{15}$-Alkylrest steht,
b) zu 0 bis 20 Gew.% aus höheren Homologen der unter a) genannten Diisocyanate mit mehr als einem Uretdionring und
c) zu 20 bis 90 Gew.% aus Diisocyanaten der Formel II

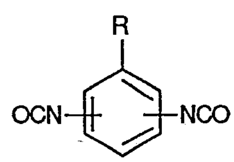 II

bestehen, wobei R die genannte Bedeutung hat, und wobei sich die Prozentsätze jeweils zu 100 ergänzen.

2. Verfahren zur Herstellung von Polyisocyanatgemischen gemäss Anspruch 1 durch Dimerisierung eines Teils der Isocyanatgruppen von aromatischen Diisocyanaten in Gegenwart von Dimerisierungskatalysatoren und Abbruch der Dimerisierungsreaktion beim jeweils gewünschten Dimerisierungsgrad durch Zugabe eines Katalysatorengifts, dadurch gekennzeichnet, dass man als zu dimerisierende Diisocyanate gegebenenfalls als Homologen- und/oder Isomerengemisch vorliegende Diisocyanate der in Anspruch 1 genannten Formel II verwendet.

3. Verwendung der Polyisocyanatgemische gemäss Anspruch 1, gegebenenfalls in Abmischung mit anderen organischen Polyisocyanaten und gegebenenfalls in mit Blockierungsmittel für Isocyanatgruppen blockierter Form als Isocyanatkomponente bei der Herstellung von Polyurethankunststoffen nach dem Isocyanat-Polyadditionsverfahren.

**Claims**

1. Polyisocyanate mixtures which are liquid at room temperature, optionally present in admixture with auxiliary agents and additives of the type used for the production of polyurethane plastics, characterised in that, based on the sum of a + b + c, they consist of
a) from 10 to 80% by weight of uretdione diisocyanates corresponding to formula I

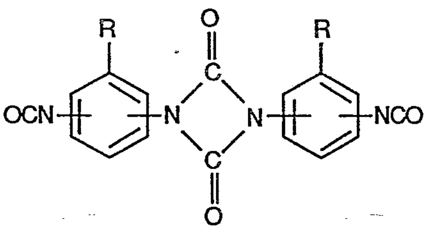

I

in which each R stands for a $C_8$-$C_{15}$-alkyl group,
b) from 0 to 20% by weight of higher homologues of the diisocyanates mentioned under a) containing more than one uretdione ring and
c) from 20 to 90% by weight of diisocyanates corresponding to formula II

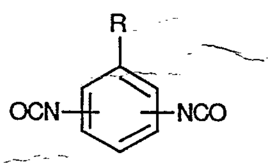

II

wherein R has the meaning indicated above, the percentages adding up to 100.

2. Process for the preparation of polyisocyanate mixtures according to claim 1 by the dimerisation of part of the isocyanate groups of aromatic diisocyanates in the presence of dimerisation catalysts and termination of the dimerisation reaction at the desired degree of dimerisation by the addition of a catalyst poison, characterised in that the diisocyanates used which are to be dimerised are diisocyanates corresponding to formula II in claim 1, optionally present as homologous and/or isomeric mixtures.

3. Use of the polyisocyanate mixtures according to Claim 1, optionally in admixture with other organic polyisocyanates and optionally blocked with blocking agents for isocyanate groups, as isocyanate components for the production of polyurethane plastics by the isocyanate polyaddition process.

**Revendications**

1. Mélanges de polyisocyanates liquides à température ambiante, éventuellement mélangés avec des produits auxiliaires et additifs du type utilisé conjointement à la préparation de résines synthétiques de polyuréthannes, caractérisés en ce qu'ils consistent, les pourcentages indiqués se rapportant à la somme a + b + c,
a) pour 10 à 80% en poids en uret-dione-diisocyanates de formule I,

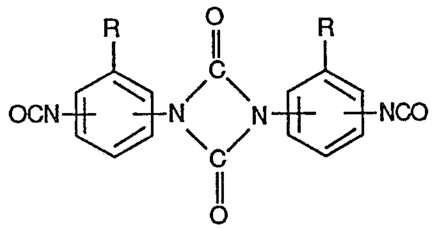

I

dans laquelle chacun des symboles R représente un groupe alkyle en $C_8$-$C_{15}$
b) pour 0 à 20% en poids en homologues supérieurs des diisocyanates mentionnés ci-dessus sous a), contenant plus d'un cycle uret-dione et
c) pour 20 à 90% en poids en diisocyanates de formule II

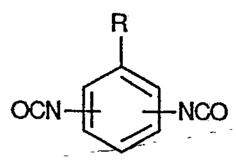

II

dans laquelle R a les significations indiquées ci-dessus, la somme des pourcentages étant égale à 100.

2. Procédé de préparation des mélanges de polyisocyanates selon la revendication 1, par dimérisation d'une partie des groupes isocyanate de diisocyanates aromatiques en présence de catalyseurs de dimérisation et interruption de la réaction de dimérisation au taux de dimérisation voulu par addition d'un poison de catalyseur, ce procédé se caractérisant en ce que l'on utilise en tant que diisocyanates à dimériser des diisocyanates de formule II de la revendication 1, éventuellement à l'état de mélanges d'homologues et/ou d'isomères.

3. Utilisation des mélanges de polyisocyanates selon la revendication 1, le cas échéant mélangés avec d'autres polyisocyanates organiques et le cas échéant à l'état bloqué par des agents bloquants des groups isocyanate, en tant que composants isocyanates à la préparation de résines synthétiques de polyuréthannes par le procédé de polyaddition des isocyanates.